# EUROPEAN PATENT APPLICATION

(11) **EP 4 600 634 A1**
(43) Date of publication of application: **13.08.2025**
(21) Application number: 24156168.7
(22) Date of filing: 06.02.2024
(51) Int. Cl.: G01N 21/15, G01N 21/43, G01N 33/44, G01N 21/85, G01N 27/22

(54) **MEASURING APPARATUS FOR MEASURING CHEMICAL OR PHYSICAL CHARACTERISTICS OF A FLUID, AND CLEANING METHOD**

(71) Applicant: Airbus Operations GmbH, 21129 Hamburg (DE)
(72) Inventor: Meyer, Tobias, 21684 Stade (DE); Schiller, Jan, 21684 Stade (DE); Teich, Benjamin, 21129 Hamburg (DE)

(57) **Abstract**

Disclosed is a measuring apparatus (1) for measuring chemical or physical characteristics of a fluid in real time, wherein a measuring window (16) of a fluid channel (4) can be cleaned by an internal cleaning device (10) having an extendable and rotatable wiper element (20), thus avoiding to open the fluid channel (4) longitudinally for cleaning purposes of the measuring window (16), a sensor carrier (2) for the measuring apparatus (1), a cleaning device (10) for the measuring apparatus (1), and a method for cleaning a measuring window (16) of a fluid channel (4).

## Description

The present invention refers to a measuring apparatus for measuring chemical or physical characteristics of a fluid, a sensor carrier for a measuring apparatus for measuring chemical or physical characteristics of a fluid, a cleaning device for a measuring apparatus for measuring chemical or physical characteristics of a fluid, and a method for cleaning a measuring window of a fluid channel.

Liquor mixtures or gas mixtures are regularly monitored regarding their chemical and/or physical characteristics. For instance, in the field of fibre composites, the mixture ratio of the resin components is checked. This can be done by feeding the resin through a fluid channel and using a sensor means. When the resin flows through the channel, contaminations/deposits can be formed on the channel wall. As a consequence, in order to avoid inaccurate measurings or readings, at least a sensor means related channel wall area has to be cleaned regularly by removing the deposits. For this purpose, the channel is opened from time to time such that it is split into two longitudinal halves and can be cleaned up. However, such cleaning is very time-consuming.

It is an object of the present invention to provide a measuring apparatus for measuring chemical or physical characteristics of a fluid, which enables a high operating time, a sensor carrier for such measuring apparatus, a cleaning device for such measuring apparatus which enables a fast cleaning of a sensor means related fluid channel wall area, and a reliable cleaning method for cleaning a sensor means related fluid channel wall area.

The object is achieved by a measuring apparatus with the features of claim 1, by a sensor carrier with the features of claim 7, by a cleaning device with the features of claim 8, and by a method with the features of claim 9. Advantageous embodiments are disclosed in the dependent claims, the description and the figures.

According to the invention, a measuring apparatus for measuring chemical or physical characteristics of a fluid, has a sensor carrier with a fluid channel inside which is adapted to feed a fluid through the sensor carrier and a sensor means which is adapted to measure at least one chemical or physical characteristic of the fluid within the fluid channel. A measuring window through which the sensor means can communicate with the fluid inside the channel forms a wall area of the fluid channel. Further on, the measuring apparatus has a cleaning device which is adapted to remove fluid deposits from the measuring window. The cleaning device has a wiper element, which is guided at least partially inside the sensor carrier opposite to the measuring window. The wiper element can be moved relative to the measuring window in at least two directions such that the wiper element touches the measuring window.

By means of this, the measuring window can be cleaned in situ in closed environments without the need of disassembly. As a consequence, the downtime of the measuring apparatus is reduced and its operating time is enhanced. Exemplary sensor means are capacitive sensors and refractometric sensors.

In a preferred embodiment, the wiper element can be moved along its longitudinal axis crossing the fluid channel and rotationally around its longitudinal axis. By moving the wiper element transversely to the fluid channel, it can be positioned in an idle position (basic/initial position) outside the fluid flow. In addition, the force under which the wiper element acts on the measuring window can be adjusted. By rotating the wiper element around its longitudinal axis, deposits on the measuring window can be removed effectively. Preferably, the wiper element and the measuring window are rotational symmetric.

In order to reach the entire measuring window just by rotating the wiper element, a front face part of the wiper element which touches the measuring window has an extension perpendicular to the longitudinal axis (rotational axis) of the wiper element is at least equal the greatest extension of the measuring window in the cross direction. In one embodiment, the front face part is a wiper blade which is fixed to a head, for instance a disk-like head, of the wiper element. In another embodiment, the front face part is a head of the wiper itself.

By making the front face part of the wiper element of a material which is softer than the material the measuring window is made of, the measuring window is resistant against wear or abrasion. For instance, the front face part is made of a rubber or elastic material. If the head is the front face part itself, the head is made of the soft material.

In order to avoid turbulences in the fluid flow which could cause inaccurate measurings, in its idle position, the wiper element forms a wall area of the fluid channel.

Alternatively, in its idle position, the wiper element rests in a recess and is covered by a cover which forms a wall area of the fluid channel.

According to the invention, a sensor carrier for a measuring apparatus for measuring chemical or physical characteristics of a fluid according to any of the preceding claims, comprises a body, a fluid channel which is formed inside the body, a body section for receiving a measuring window forming a wall area of the fluid channel, and a body section for receiving at least partially a cleaning device which is positioned opposite to the body section for receiving the measuring window.

Such a sensor carrier enables a cleaning of the measuring window without being disassembled. Consequently, the sensor carrier could be a single part as an opening of the fluid channel is not needed for cleaning the measuring window. However, for measuring reasons of the fluid channel at a whole, the sensor carrier may have a lower half and an upper half, whose separation plane runs through the fluid channel in longitudinal direction.

According to the invention, a cleaning device is adapted to be installed in an inventive measuring apparatus and/or to be installed in an inventive sensor carrier.

According to the invention, a method for cleaning a measuring window of a fluid channel, wherein the measuring window forms a wall part of a fluid channel inside a sensor carrier, comprises the steps:
- Extending a wiper element of a cleaning device from an idle position inside the sensor carrier in a working position, in which it touches the measuring window of the sensor means;
- Moving the wiper element on the measuring window until fluid deposits are removed from the measuring window; and
- Retracting the wiper element back in its idle position;
Such cleaning method enables a reliable and fast cleaning of the measuring window.

Preferably, cleaning parameters such as a pressure force, rotational speed, rotational direction of the wiper element, and/or duration of the cleaning process can be adjusted. By adjusting the cleaning parameters individually, the deposits can be removed optimally from the measuring window.

In what follows, preferred embodiments of the present invention are explained with respect to the accompanying drawings. As is to be understood, the various elements and components are depicted as examples only, may be facultative and/or combined in a manner different than that depicted. Reference signs for related elements are used comprehensively and not necessarily defined again for each figure. Shown is schematically in
- Figure 1: a longitudinal section of an inventive measuring apparatus before cleaning its measuring window;
- Figure 2: a longitudinal section of an inventive measuring apparatus during cleaning the measuring window;
- Figure 3: a longitudinal section of an inventive measuring apparatus after cleaning the measuring window;
- Figure 4: a longitudinal section of an alternative inventive measuring apparatus during cleaning the measuring window;
- Figure 5: an inventive cleaning device with its wiper element in a retracted state;
- Figure 6: the cleaning device with its wiper element in an extended state; and
- Figure 7: a front view of an exemplary head of the wiper element.

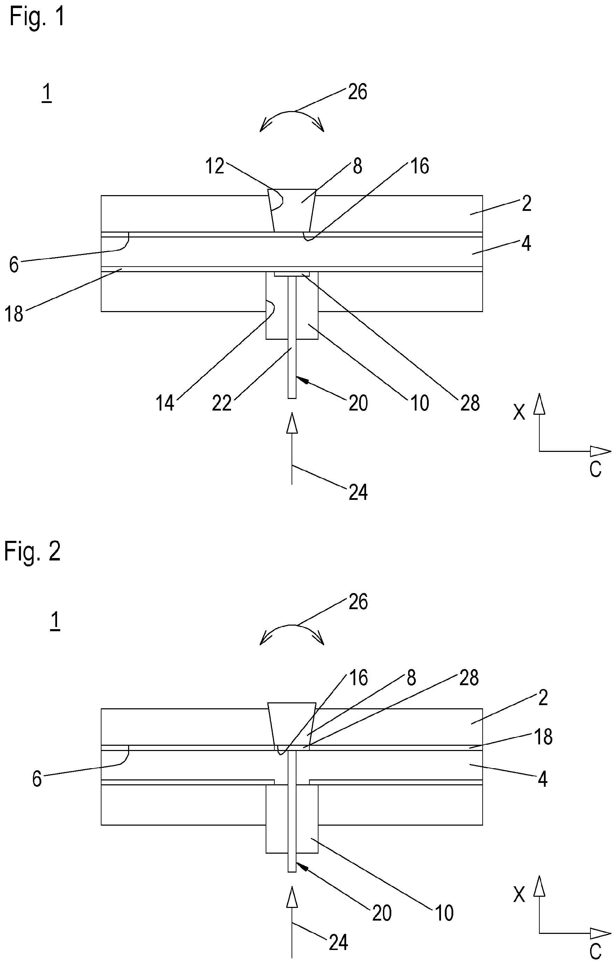

In Figure 1, a first embodiment of a measuring apparatus 1 according to the invention is shown. The measuring apparatus 1 can be used for measuring at least one chemical or physical characteristics of a fluid (liquor of gas) in real time. An exemplary fluid is a resin used for fibre composites, such as CFRP or GFRP. The characteristic which should be measured is the mixture ratio of the resin. The measuring apparatus can be an integral part of a forming/molding tooling or separate part of such tooling.

The measuring apparatus 1 has a sensor carrier 2 in which a fluid channel 4 is provided. The fluid channel 4 extends through the sensor carrier 2 and has preferably a rectangular-like cross section, which is delimited by its channel wall 6. However, the fluid channel 4 can also have a different cross section such as a circular cross section. It is also possible, that the cross section of the fluid channel varies. For instance, along a measuring path, the cross section can be rectangular and in a channel area before and/or after the measuring path, the fluid channel 4 can has a circular cross section.

The fluid channel 4 is provided to feed a fluid to be monitored through the sensor carrier 2. For this purpose, the sensor carrier 2 can be integrated into a not shown fluid system, feeding the fluid to the fluid channel 4 and discharging the fluid from the fluid channel afterwards.

In order to measure the at least one fluid characteristic, the measuring apparatus 1 comprises a sensor means 8. Further on, the measuring apparatus 1 comprises a cleaning device 10. Both, the sensor means 8 and the cleaning device 10 are positioned at least partially in a recess 12, 14 of the sensor carrier 2.

In this example, the sensor means 8 is a capacitive sensor. A front measuring surface of the sensor builds a part of the fluid channel wall 6 and acts as a measuring window 16 of the fluid channel 4. For instance, the measuring surface, and such the measuring window 16, can be a metallic, flat surface.

The cleaning device 10 is positioned in its carrier recess 14 opposite to the sensor means 8. It is used to remove contaminations/deposits 18 of the fluid from the measuring window 16, preferably before a new measurement series starts. The cleaning device 10 has a wiper element 20 which is guided inside a not shown bearing or bushing arrangement and whose movable and rotatable shaft 22 is sealed against fluid leakage.

The wiper element 20 can be moved in two directions. The first direction 24 corresponds to its longitudinal axis x and the second direction corresponds to a rotation 26 around the longitudinal axis x. The longitudinal axis x of the wiper element 20 extends orthogonally to the measuring window 16 and orthogonally to a central axis c of the fluid channel 4. In order to enable a rotation of the wiper element 20 on the measuring window 16, both a head 28 of the wiper element 20 touching the measuring window 16 for cleaning reasons and the measuring window 16 are rotationally symmetrically.

If not in use, in particular during a measurement, the wiper element 20 rests in an idle position. In its basis position the wiper element 20 is positioned outside of a fluid flow. In this example, the head 28 of the wiper element 20 forms an area of the channel wall 6. As shown in Figure 1, the head 28 of the wiper element 20 is also contaminated.

Before a new measurement series starts, all contamination/deposits 18 will be removed from the measuring window 16. For this purpose, as shown in Figure 2, the wiper element 20 extends from its idle position and is moved along its longitudinal axis x across the fluid channel 4 in its working position. In its working position, its head 28 touches the measuring window 16 and rotates around its longitudinal axis x. In order to achieve an optimal cleaning result, a pressure force, rotational speed, rotational direction and/or duration of the cleaning process can be adjusted as a function of the kind of contamination/deposits 18. As a consequence, the contamination 18 on the measuring window 16 and on the head 28 are removed (wiped off).

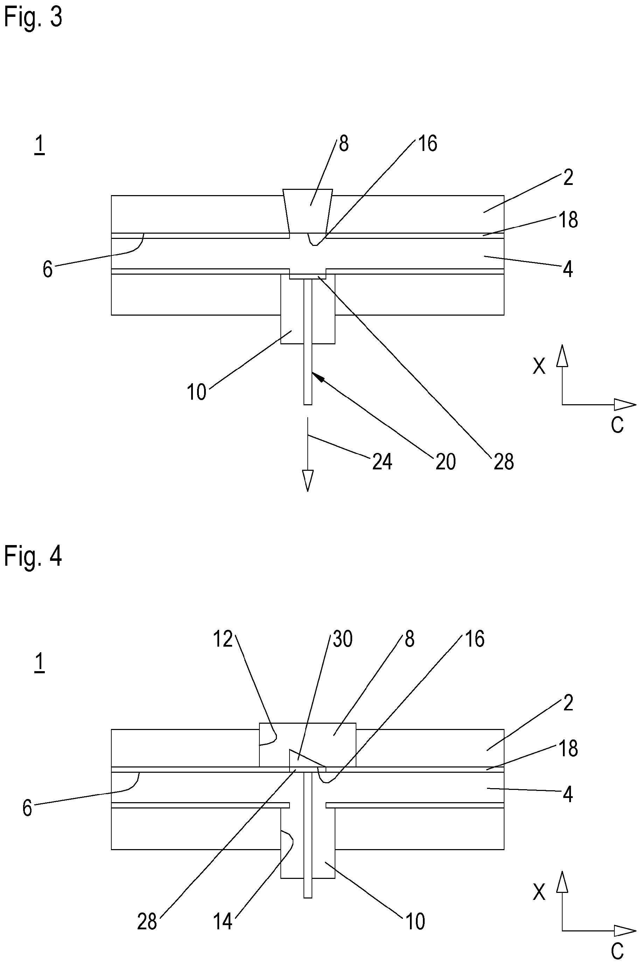

After the cleaning of the measuring window 16 is terminated, the rotation is stopped and the wiper element 20 is retracted back into its idle position, as shown in Figure 3. Now, the measuring apparatus is prepared for a new measurement.

In Figure 4, an alternative measuring apparatus 1 is shown in a working position of a wiper element 20. Contrary to the measuring apparatus 1 described in Figures 1, 2 and 3, the alternative measuring apparatus 1 is equipped with a sensor means 8 based on refractometry (refractometric sensor). For example, a prism 30 is provided via whom light waves are emitted into a fluid flow and received back. Here, a boundary layer of the prism 30 to the fluid channel 4 forms the measuring window 16 of the fluid channel 4.

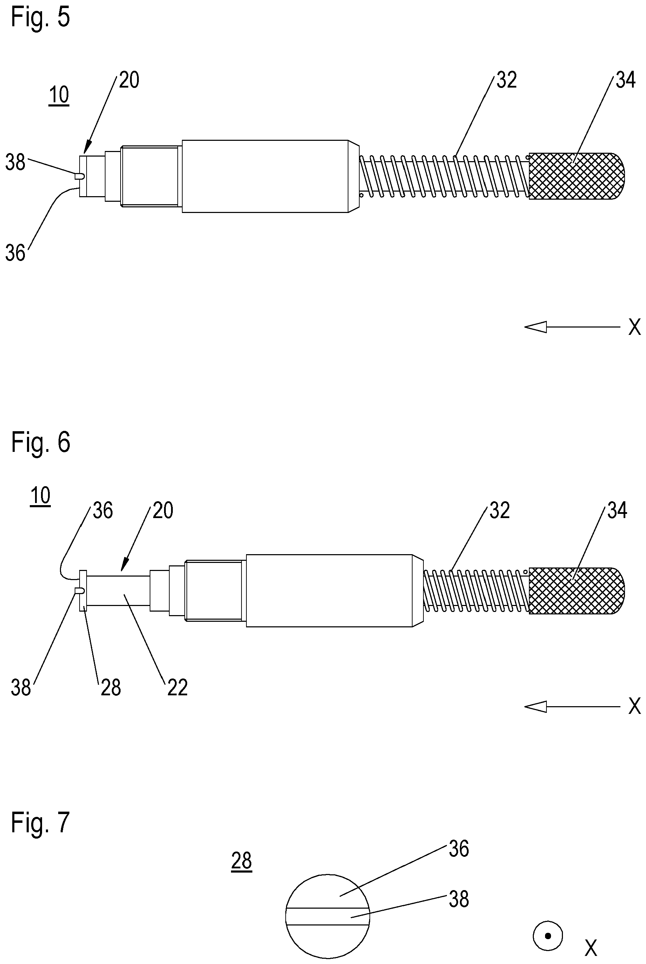

In Figure 5 and 6, an exemplary cleaning device 10 is shown in its idle position (Figure 5) and its working position (Figure 6). In the idle position, the wiper element 20 is retracted. In the working position, the wiper element 20 is extended. In order to facilitate the retraction movement, on a backside of the cleaning element 10, a spring 32 is provided by means of which the wiper element 20 is preloaded in its idle position. In addition, a rear handle 34 can be provided in order to operate the cleaning device 10 manually. In a preferred mode, the cleaning device 10 is operated fully automatically.

As also illustrated in more detail in Figures 5, 6 and 7, the head 28 of the wiper element 20 has a disc-like shape and comprises at its front face 36 a wiper blade. 38 The wiper blade 38 has a longitudinal shape and extends through a disc centre radially on the front face 36 of the head 28. In particular, the wiper blade 38 has an extension transversely to the longitudinal axis a (rotational axis) of the wiper element 20 that is at least equal to the greatest extension of the measuring window 16 in the cross direction. The wiper blade 38 is held in a groove of the head 28 and made of a material which is softer than the material of the measuring window 16. For instance, the wiper blade 38 is made of an elastic or rubber like material whereas the measuring window is made of glass. Thereby, it is important that during a cleaning process the wiper blade 38 does not cause any streaks, smudges or similar effects on the measuring window 16, which could have a negative impact on the measurement result. If the wiper blade 38 is worn-out, it will be replaced by a new one. Then, the cleaning mean 10 will be removed from the sensor carrier 2 backwards and inserted into the recess after the wiper blade 38 has been renewed. When removing and replacing the used cleaning device 10, an assembled state of the sensor carrier 2 can be maintained. Both, the cleaning device 10 and the sensor means 8 can be hold in place via a screw connection, a snap- and click connection and the like.

As the wiper blade 38 extends over the front face 34 of the head 28 in longitudinal direction x of the wiper element 20, the head 28 always touches the measuring window 16 via the wiper blade 38.

Disclosed is a measuring apparatus for measuring chemical or physical characteristics of a fluid in real time, wherein a measuring window of a fluid channel can be cleaned by an internal cleaning device having an extendable and rotatable wiper element, thus avoiding to open a fluid channel longitudinally for cleaning purposes of the measuring window, a sensor carrier for the measuring apparatus and a cleaning method.

### Reference list

- 1: measuring apparatus
- 2: sensor carrier
- 4: fluid channel
- 6: channel wall
- 8: sensor means
- 10: cleaning device
- 12: recess for sensor means / body section
- 14: recess for cleaning device / body section
- 16: measuring window
- 18: contamination / deposits
- 20: wiper element
- 22: shaft
- 24: first direction
- 26: second direction
- 28: head of the wiper element
- 30: prism
- 32: spring
- 34: handle
- 36: front face
- 38: wiper blade / front face part

- x: longitudinal axis of the fluid channel
- c: central axis (rotational axis) of the wiper element

## Claims

1. A measuring apparatus (1) for measuring chemical or physical characteristics of a fluid, comprising
- a sensor carrier (2) having a fluid channel (4) adapted to feed a fluid through the sensor carrier (2);
- a measuring window (16) forming a wall area of the fluid channel (4);
- a sensor means (8) adapted to measure at least one chemical or physical characteristic of the fluid within the fluid channel (4) through the measuring window (16),
and
- a cleaning device (10) adapted to remove fluid deposits (18) from the measuring window (16),
wherein the cleaning device (10) has a wiper element (20), which is guided at least partially inside the sensor carrier (2) opposite to the measuring window (16),
wherein the wiper element (20) can be moved relative to the measuring window (16) in at least two directions (24, 26) such that the wiper element (20) touches the measuring window (16).

2. The measuring apparatus (1) according to claim 1, wherein the wiper element (20) can be moved along its longitudinal axis (x) crossing the fluid channel (4) and rotationally around its longitudinal axis (x).

3. The measuring apparatus (1) according to claims 1 or 2, wherein a front face part (38) of the wiper element (20) touching the measuring window (16) has an extension cross to the longitudinal axis (x) of the wiper element (20) that is at least equal to the greatest extension of the measuring window (16) in its transversal direction.

4. The measuring apparatus (1) according to claims 1, 2 or 3, wherein the wiper element (20) has a front face part (38) made of a material which is softer than the material the measuring window (16) is made of.

5. The measuring apparatus (1) according to any of the preceding claims, wherein in its idle position, the wiper element (20) forms a wall area of the fluid channel (6).

6. The measuring apparatus (1) according to any of claims 1 to 4, wherein in its idle position, the wiper element (20) is covered by a cover which forms a wall area of the fluid channel (6).

7. A sensor carrier for a measuring apparatus (1) for measuring chemical or physical characteristics of a fluid according to any of the preceding claims, comprising:
- a body,
- a fluid channel (4) formed inside the body;
- a body section (12) for receiving a measuring window (16) forming a wall area of the fluid channel (4); and
- an opposite body section (14) for receiving at least partially a cleaning device (10).

8. A cleaning device adapted to be installed in a measuring apparatus (1) for measuring chemical or physical characteristics of a fluid according to any of the preceding claims 1 to 6 and/or to be installed in a sensor carrier according to claim 7.

9. A method for cleaning a measuring window (16) of a fluid channel (4), wherein the measuring window (16) forms a wall part of a fluid channel (4) inside a sensor carrier,
comprising the steps:
- Extending a wiper element (20) of a cleaning device (10) from a idle position inside the sensor carrier (2) in a working position, in which it touches the measuring window (16) of the sensor means (8) ;
- Moving the wiper element (20) on the measuring window (16) until fluid deposits (18) are removed from the measuring window (16); and
- Retracting the wiper element (20) back in its idle position;

10. The method according to claim 9, wherein a pressure force, rotational speed, rotational direction and/or duration of the cleaning process are adjusted.
